# EUROPÄISCHE PATENTSCHRIFT

(11) **EP 0 462 062 B1**
(45) Veröffentlichungstag und Bekanntmachung des Hinweises auf die Patenterteilung: **09.11.1994**
(21) Anmeldenummer: 91810424.1
(22) Anmeldetag: 04.06.1991
(51) Int. Cl.: B29C 35/02, B29C 43/58, G01N 33/00

(54) **Verfahren zum Überprüfen des Gelierprozesses nach dem Giessen eines reaktiven Harzsystems in eine Produktionsform**
Method of testing the gelling process following casting of a reactive resin in a production mould
Procédé pour tester la gélification après le coulage d'une résine réactive dans un moule de production

(30) Priorität: 13.06.1990 CH 1986/90; 27.08.1990 CH 2764/90
(43) Veröffentlichungstag der Anmeldung: 18.12.1991
(73) Patentinhaber: CIBA-GEIGY AG, 4002 Basel (CH)
(72) Erfinder: Munk, Kurt, Dr., W-7889 Grenzach (DE); Heizler, Jürg, CH-4054 Basel (CH)

(56) Entgegenhaltungen:
- EP-A- 0 259 252
- WO-A-91/04487
- GB-A- 798 573
- US-A- 4 810 438
- US-A- 4 828 472
- WORLD PATENTS INDEX LATEST, AN=84-149677 [24], Woche 8424, Derwent Publications Ltd, London, GB; & JP-A-59 078 988 (MITSUI KENSETSU)

## Beschreibung

Die Erfindung betrifft ein Verfahren zum Überprüfen des Gelierprozesses nach dem Gießen eines reaktiven Harzsystems in eine Produktionsform.

Das Prinzip des Druckgelierverfahrens ist aus Anwendungsbeispiele für Araldit, Nr. 39, "Das Druckgelierverfahren - eine rationelle Giesstechnik zur Herstellung von Epoxidharz-Formstoffen" der Ciba-Geigy AG, Division Kunststoffe und Additive, Basel, Mai 1972 bekannt. Beim Aushärten eines reaktiven Harzsystems erfolgt während des Gelierprozesses eine mit einem Temperaturanstieg verbundene exotherme chemische Reaktion sowie ein Reaktionsschwund des Volumens der aushärtenden Masse, so daß in Abhängigkeit vom räumlichen Fortschreiten des Gelierprozesses entlang einer oder mehrerer Gelierfronten neben der Gefahr einer ungleichmäßigen Gelierung sowie dadurch bedingter lokaler Überhitzungen die Gefahr besteht, daß durch die starke Volumenabnahme beim Erstarren Lunker gebildet werden, wenn Außenzonen eines Volumens vollständig erstarrt sind, aber in dessen Kernzone noch nicht erstarrtes Harz vorliegt. Derartige Fehler können trotz der guten Steuerung und Kontrolle des Reaktionsablaufs beim Druckgelierverfahren vorkommen, wenn die Produktionsform geometrisch oder thermisch ungünstig ausgebildet ist.

Man hat bereits versucht, das Fortschreiten der Erstarrungsfront von schnell aushärtenden Epoxidharzen und ähnlichen Materialien mit Hilfe eines Computerprogramms zu simulieren (Erich Knauder, Diplomarbeit, Montanuniversität Leoben, Österreich, 16. Februar 1989). Dieses sowie andere Computerprogramme liefern aber Ergebnisse, die den Genauigkeitsanforderungen in der Praxis noch nicht gerecht werden.

Es ist auch bereits bekannt, zur Ermittlung des zeitlichen Gelierverhaltens eines reaktiven Harzsystems mechanische Gelierpunktsonden zu verwenden, bei denen ein im Harz eingetauchter und bis zum Aushärten auf- und abbewegter Stift beim Erreichen des Gelierpunktes ein Signal auslöst (TECAM-Meßgerät). Beim Verwenden eines derartigen Meßgerätes zum Überprüfen des Gelierprozesses in einer Produktionsform werden jedoch Störeinflüsse erzeugt, so daß nicht mehr der in Wirklichkeit vorhandene Verlauf der Gelierfront oder Erstarrungsfront in der Produktionsform erfaßt werden kann.

Der Erfindung liegt die Aufgabe zugrunde, ein Verfahren zu schaffen, das es gestattet, das räumliche Wandern der Gelierfront während des Härtungsablaufs in einer zum Herstellen einer Vielzahl von Gießlingen vorgesehenen Produktionsform als Funktion der Zeit mit hoher Genauigkeit dreidimensional zu erfassen.

Diese Aufgabe wird erfindungsgemäß dadurch gelöst, daß während einer Kalibriermessung das Gelierverhalten des für die Herstellung des Gießlings vorgesehenen Harzsystems in einer auf verschiedene vorherbestimmte Temperaturen aufheizbaren Kalibrierform ermittelt wird, indem bei verschiedenen Kalibrierformtemperaturen für mehrere unterschiedliche Gelierverhalten aufweisende Volumenelemente innerhalb der Kalibrierform nach dem Einbringen des Harzsystems in die Kalibrierform mittels Gelierpunktsonden der Gelierzeitpunkt und mittels Thermofühler die zugeordneten zeitlichen Temperaturverläufe vom Zeitpunkt des Einbringens des Harzsystems in die Kalibrierform bis zur jeweiligen nach dem Gelierzeitpunkt auftretenden maximalen Temperatur bei der Exothermiespitze erfaßt werden, daß die ermittelten jeweiligen Gelierzeitpunkttemperaturen abgespeichert werden und für jeden Temperaturverlauf eine zugeordnete charakteristische skalare Größe bestimmt wird, indem das Kalibriervolumen eines zugeordneten Vergleichskörpers ermittelt und gespeichert wird, dessen Grundfläche durch den zeitlichen Temperaturverlauf zwischen der minimalen und der maximalen Temperatur des Harzsystems in der Kalibrierform und dessen Höhe durch einen sich in Richtung der Temperaturachse des Temperaturverlaufs verändernden Gelierfaktor bestimmt ist, der dem Kehrwert der Gelierzeit bei der jeweiligen Temperatur entspricht, daß während einer Überprüfungsmessung unter Produktionsbedingungen in der vorgesehenen Produktionsform durch eingießbare Thermofühler die zeitlichen Temperaturverläufe des bei der Kalibrierung verwendeten Harzsystems an mehreren unterschiedlichen Meßstellen innerhalb der Produktionsform erfaßt und gespeichert werden, daß für die so ermittelten Temperaturverläufe zwischen den minimalen und maximalen Temperaturen des Harzsystems an den jeweiligen Meßstellen die Meßvolumen zugeordneter Vergleichskörper ermittelt werden, deren Grundfläche durch den zeitlichen Temperaturverlauf zwischen der minimalen und der maximalen Temperatur des Harzsystems in der Produktionsform und dessen Höhe durch den Gelierzeitfaktor bestimmt ist, daß die für die unterschiedlichen Meßstellen innerhalb der Produktionsform ermittelten Meßvolumen der Vergleichskörper mit den zusammen mit den Gelierzeitpunkttemperaturen abgespeicherten Kalibriervolumen der Vergleichskörper verglichen werden und daß für die unterschiedlichen Meßstellen in der überprüften Produktionsform durch eine Interpolation der Meßvolumen zwischen den gespeicherten Kalibriervolumen die zugeordneten interpolierten individuellen Gelierzeitpunkttemperaturen ermittelt werden, aus denen sich ausgehend von den gespeicherten räumlich verteilt erfaßten zeitlichen Temperaturverläufen und dem sich aus diesen ergebenden Verlauf der Isothermen für jeden Zeitpunkt der Verlauf der die Isothermen schneidenden Gelierfront in der Produktionsform ergibt.

Dadurch, daß das Durchziehen einer Gelierfront nicht mehr mechanisch sondern gemäß der Erfindung thermisch erfaßt wird, ist es möglich, mit Hilfe von in einer zu überprüfenden Produktionsform räumlich verteilt angeordneten Thermoelementen nicht nur das thermische Geschehen zeitlich und räumlich zu erfassen, sondern darüber hinaus aus der zeitlichen Wanderung von Isothermen unter der Berücksichtigung gemessener Gelierzeitpunkttemperaturen das räumliche Fortschreiten von Gelierfronten und damit den Härtungsablauf in einer tatsächlichen Produktionsform. Die in der Produktionsform an verschiedenen Stellen unterzubringenden Thermoelemente sind über dünne, weder das Gießen noch das Aushärten beeinträchtigende Drähte mit einer Einrichtung zum Erfassen, Speichern und Auswerten zeitlicher Temperaturverläufe verbunden.

Nachfolgend wird ein Ausführungsbeispiel der Erfindung anhand der Zeichnung näher beschrieben. Es zeigen:
- Fig. 1: einen Längsschnitt durch eine Kalibrierform zur Durchführung von Kalibriermessungen an Harzsystemen bei verschiedenen Temperaturen,
- Fig. 2: von an sechs verschiedenen Stellen in der Kalibrierform angeordneten Thermofühlern erfaßte Temperaturverläufe vom Zeitpunkt des Einfüllens des Harzsystems in die Kalibrierform bis zu einem nach der Exothermiespitze liegenden Zeitpunkt,
- Fig. 3: eine schematische Darstellung eines einzelnen Temperaturverlaufs entsprechend Fig. 2,
- Fig. 4: die Gelierzeit eines Harzsystems als Funktion der Temperatur des Harzsystems,
- Fig. 5: den Verlauf eines Gelierzeitfaktors, der dem Kehrwert der Gelierzeit bei der jeweiligen Temperatur entspricht,
- Fig. 6: den Verlauf eines angenähert berechneten Gelierzeitfaktors in Abhängigkeit von der Temperatur und
- Fig. 7: eine schematische Darstellung einer heizbaren Gießform zur Veranschaulichung der räumlichen Anordnung einer Vielzahl von Thermofühlern während einer Überprüfungsmessung in einer Produktionsform.

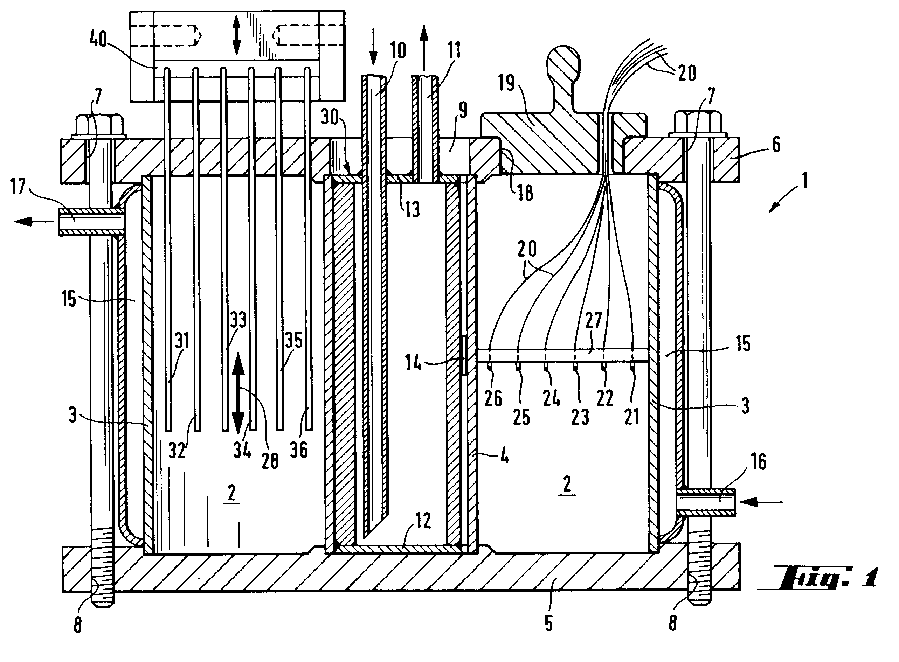

In Fig. 1 erkennt man, einen Längsschnitt durch eine Kalibrierform 1 zur Durchführung von Kalibriermessungen an Harzsystemen bei verschiedenen Temperaturen. Die Kalibrierform 1 hat eine rotationssymmetrische vorgeheizte Formkavität 2, in die eine aufbereitete Gießharzmischung, insbesondere ein Harzsystem auf Epoxidharzbasis mit einem Quarzmehl als Füllstoff eingefüllt werden kann. Die Formkavität 2, die in Fig. 1 im Längsschnitt dargestellt ist, hat im Querschnitt rechtwinklig zur Ebene der Fig. 1 eine ringförmige Gestalt, wobei die Formkavität 2 entlang dem größeren Umfang durch einen zylindrischen Außenmantel 3 und entlang dem kleineren Umfang durch einen zylindrischen Innenmantel 4 begrenzt ist. Die durch Rohrstücke gebildeten zylindrischen Mäntel 3 und 4 sind durch eine Bodenplatte 5 verschlossen.

Auf der, der Bodenplatte 5 gegenüberliegenden Seite, ist die ringförmige Formkavität 2 durch eine Deckplatte 6 verschlossen. In der in Draufsicht kreisrunden Deckplatte 6 befinden sich entlang dem Umfang verteilt Befestigungslöcher 7, durch die Befestigungsschrauben steckbar sind, die mit ihrem vorderen Ende in zugeordnete Befestigungsgewinde 8 in der Bodenplatte 5 einschraubbar sind und mit ihren Köpfen die Deckplatte 6 und die Bodenplatte 5 zusammenhalten.

In der runden Deckplatte 6 befindet sich weiterhin eine konzentrische Öffnung 9 durch die ein Öleinlaßrohr 10 und ein Ölauslaßrohr 11 in eine im Inneren des zylindrischen Innenmantels 4 angeordnete zylindrische Heizpatrone 30 hineinragen, die an ihrer Unterseite durch eine Bodenscheibe 12 und an ihrer Oberseite durch eine Abdeckscheibe 13 verschlossen ist.

Durch das Öleinlaßrohr 10, dessen vorderes Ende bis in die Nähe der Bodenscheibe 12 der Heizpatrone 30 hinunterragt, wird auf eine vorbestimmte Temperatur aufgeheiztes Öl zum Aufheizen der Kalibrierform 1 in die Heizpatrone 30 eingebracht. Das abgekühlte Öl verlaßt die den zylindrischen Innenmantel 4 von dessen Innenseite her berührende Heizpatrone 30 durch das Ölauslaßrohr 11. Ein in einer Fase des zylindrischen Gehäuses der Heizpatrone 30 angeordneter Temperaturfühler 14 gestattet eine Temperaturerfassung an der Heizpatrone 30.

Der zylindrische Außenmantel 3 ist von einem Heizmantel 15 umgeben, so daß zwischen dem Heizmantel 15 und dem zylindrischen Außenmantel 3 ein Ringraum gebildet ist, der ebenfalls zum Aufheizen der Formkavität 2 von heißem Öl durchströmt werden kann. Das zum Aufheizen verwendete Öl gelangt durch einen Eintrittsstutzen 16 in den Zwischenraum zwischen dem Heizmantel 15 und dem Außenmantel 3 ein und verläßt diesen durch einen Austrittsstutzen 17. Die Heizpatrone 30 und der Heizmantel 15 gestatten es, die Kalibrierform 1 auf verschiedene vorherbestimmte Temperaturen zu Kalibrierzwecken aufzuheizen.

Um ein Harzsystem wie Epoxidharz oder Araldit in die Formkavität 2 einfüllen zu können, ist in der Deckplatte 6 eine in Fig. 1 im Schnitt erkennbare Einfüllöffnung 18 vorgesehen, die durch eine Abdeckung 19 verschließbar ist.

Durch die Abdeckung 19 verlaufen eine Vielzahl von Anschlußdrähten 20, die jeweils paarweise an ihren vorderen Enden miteinander zu Thermoelementen 21,22,23,24,25 und 26 verbunden sind. Die Thermoelemente 21 bis 26 sind durch einen Distanzhalter 27 entlang einem Radius der Formkavität 2 in axialer Richtung etwa in der axialen Mitte der Formkavität 2 fixiert und gestatten das Erfassen von Temperaturverläufen an den jeweiligen Stellen innerhalb der Formkavität 2 und damit im Innern eines aushärtenden Harzsystems vom Zeitpunkt des Einbringens des Harzsystems in die als Kalibrierform dienende Formkavität 2 bis zur jeweiligen nach dem Gelierzeitpunkt auftretenden maximalen Temperatur bei der Exothermiespitze.

Den Thermoelementen 21 bis 26 diametral gegenüberliegend sind in Richtung des Doppelpfeils 28 auf- und abbewegbare Glasröhrchen 31 bis 36 angeordnet. Die Glasröhrchen 31 bis 36 und die Thermoelemente 21 bis 26 liegen dabei jeweils auf konzentrischen Kreisen um den Innenmantel 4 innerhalb einer auf der Zeichenebene rechtwinklig stehenden Querschnittsebene durch die Kalibrierform 1. Wegen der Rotationssymmetrie der Kalibrierform 1 sind somit die physikalischen und insbesondere die thermischen Verhältnisse am Ort des Thermoelementes 21 identisch mit denjenigen am Ort des Glasröhrchens 31. Entsprechendes gilt für das Thermoelement 22 und das Glasröhrchen 32 sowie die übrigen Thermoelemente 23 bis 26 und Glasröhrchen 33 bis 36. Auf diese Weise gestatten die von den Glasröhrchen 31 bis 36 räumlich getrennten Thermoelemente 21 bis 26 eine genaue Erfassung der Temperaturen an den Stellen der Glasröhrchen 31 bis 36 zur Gelierzeitpunkttemperaturbestimmung.

Die in das Harzsystem eingetauchten Enden der Glasröhrchen 31 bis 36 beenden die Auf- und Abbewegung wenn das Harzsystem, in das sie eintauchen an den jeweiligen Stellen aushärtet, wobei die Glasröhrchen 31 bis 36 in der Nähe einer Halterung 40 abbrechen. In Fig. 1 erkennt man die Halterung 40 für die Glasröhrchen 31 bis 36, die es einerseits gestattet, den Glasröhrchen 31 bis 36 die zur Gelierzeitmessung erforderliche vertikale Bewegung zu verleihen, und die andererseits das Abbrechen eines Glasröhrchens 31 bis 36 beim Durchqueren der Gelierfront mit Hilfe einer nicht gezeichneten Kontakteinrichtung zu erfassen gestattet. Wenn somit beispielsweise die Gelierfront in radialer Richtung das Glasröhrchen 31 durchquert und dieses vom aushärtenden Harz festgehalten wird, wird mit Hilfe der Halterung 40 ein entsprechendes Signal ausgelöst, so daß dem so erfaßten Gelierzeitpunkt als Gelierzeitpunkttemperatur die vom symmetrisch angeordneten Thermoelement 21 gerade erfaßte Gelierzeittemperatur zugeordnet werden kann.

Die Kalibrierform 1 mit den durch die Glasröhrchen 31 bis 36 sowie der zugehörigen Mechanik und Auswerteeinrichtung gebildeten Gelierpunktsonden gestatten es somit, zusammen mit den Thermoelementen 21 bis 26 an unterschiedlichen Stellen innerhalb der Formkavität 2 für unterschiedliche Öltemperaturen Temperaturverläufe zu erfassen und für jeden erfaßten Temperaturverlauf genau die Gelierzeitpunkttemperatur an unterschiedlichen Stellen, d.h. für mehrere Volumenelemente in der Kalibrierform 1, zu ermitteln. Derartige Kalibriermessungen ergeben jeweils für ein bestimmtes Harzsystem Kalibrierdaten, die es später gestatten, in einer beliebigen Produktionsform die zeitliche Wanderung der Gelierfront lediglich durch Temperaturmessungen zu bestimmen.

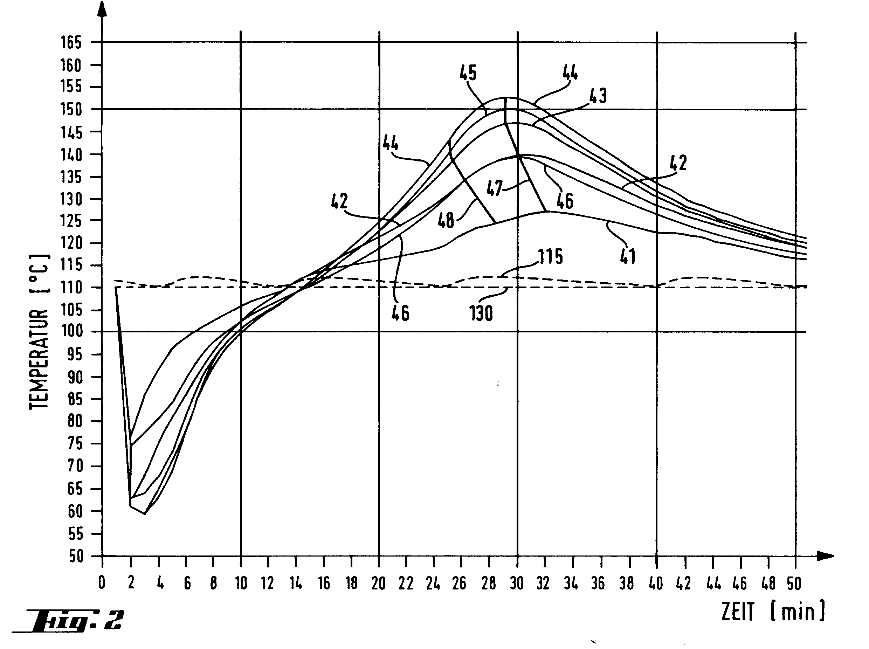

In Fig. 2 erkennt man ein Beispiel für sechs Temperaturverläufe 41 bis 46, die mit Hilfe der Thermoelemente 21 bis 26 für ein spezielles Harzsystem bei einer Temperatur der Kalibrierform 1 von 110° C erfaßt worden sind. Der Verlauf der geregelten Heizmanteltemperatur ist mit dem Bezugszeichen 115 und der Verlauf der Heizpatronentemperatur mit dem Bezugszeichen 130 versehen. Wie man in Fig. 2 erkennt, sinkt die Temperatur an den Stellen der Thermoelemente 21 bis 26 innerhalb von etwa zwei Minuten nach dem Einfüllen des Harzsystemes auf Werte im Bereich von 60° bis 80° C ab und steigt dann durch Aufheizen des ursprünglich kalten Harzsystems sowie durch die beim Aushärten freiwerdende Wärmetönung bis jeweils zu maximalen Temperaturen oder Exothermiespitzen an, die in Fig. 2 durch eine Exothermielinie 47 miteinander verbunden sind.

Die unter Verwendung der Glasröhrchen 31 bis 36 gebildeten Gelierpunktsonden gestatten das Erfassen und Markieren der Gelierzeitpunkte auf den Temperaturverläufen 41 bis 46. In Fig. 2 sind die Gelierzeitpunkte durch eine Gelierzeitpunktlinie 48 miteinander verbunden. Man erkennt, daß die Gelierfront an den Kalibriermeßstellen der Thermoelemente 21 bis 26 und der Glasröhrchen 31 bis 36 innerhalb eines Zeitraumes von etwa 25 bis 28 Minuten nach dem Einfüllen des kalten Harzsystems durchzieht. Die Exothermiefront tritt je nach der radialen Lage des betrachteten Volumenelementes um eines der Thermoelemente 21 bis 26 erst nach etwa 29 bis 32 Minuten auf, wie der Fig. 2 entnommen werden kann. Die zugehörigen Gelierzeitpunkttemperaturen und Exothermiespitzentemperaturen können der Fig. 2 ebenfalls entnommen werden und liegen je nach dem vermessenen Volumenelement etwa zwischen 124° und 153° C.

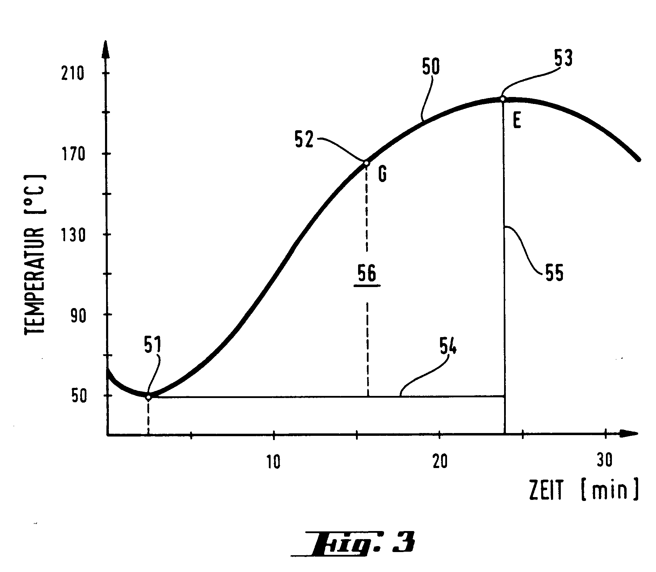

Fig. 3 zeigt stark schematisiert und beispielhaft einen Temperaturverlauf 50 wie er aus Fig. 2 bekannt ist und mit Hilfe der Thermoelemente 21 bis 26 für ein spezielles Volumenelement erfaßt werden kann. Der Temperaturverlauf 50 veranschaulicht den zeitlichen Verlauf der von einem der Thermoelemente 21 bis 26 in der Kalibrierform 1 gemessenen Temperatur eines Harzsystems, das die vorgeheizte Kalibrierform 1 nach dem Einfüllen zunächst abkühlt, bis eine Minimaltemperatur 51 nach etwa 2 bis 3 Minuten bei dem in der Fig. 3 dargestellten Beispiel erreicht ist. Beim Erreichen der Minimaltemperatur 51 beginnt die insbesondere durch exotherme Erwärmung verursachte Temperaturerhöhung des in die Kalibrierform 1 eingefüllten Harzsystems. Bei der exothermen Erwärmung steigt die von einem der Thermoelemente 21 bis 26 erfaßte Temperatur in der aus Fig. 3 ersichtlichen Weise an, bis für das jeweilige Volumenelement der durch das Bezugszeichen 52 gekennzeichnete Gelierpunkt G erreicht und gemessen wird. Beim Erreichen des Gelierpunktes 52 wird mit Hilfe der die Glasröhrchen 31 bis 36 enthaltenden Gelierpunktsonde der Gelierpunkt 52 zeitlich erfaßt. In Fig. 3 erkennt man, daß bei dem dort dargestellten Beispiel der Gelierpunkt nach etwa 16 Minuten bei einer Temperatur von etwa 170° erreicht wird. Die Koordinaten des Gelierpunktes 52 in dem in Fig. 3 dargestellten Koordinatensystem liefern den Gelierzeitpunkt und die Gelierzeitpunkttemperatur für ein Volumenelement um das jeweilige Thermoelement eines Harzsystems.

Während einer Kalibriermessung unter Verwendung der Kalibrierform 1 wird der Temperaturverlauf 50 in einer in der Zeichnung nicht dargestellten Auswerteeinheit₇ z.B. einen Computer abgespeichert. Der Computer erhält über die dem jeweiligen Thermoelement 21 bis 26 zugeordnete Gelierpunktsonde mit den Glasröhrchen 31 bis 36 beim Erreichen des Gelierpunktes 52 ein Signal, so daß neben dem Temperaturverlauf 50 auch die Koordinaten des mit dem Bezugszeichen 52 in Fig. 3 versehenen Gelierpunktes G gespeichert werden können. Von besonderer Bedeutung für die weitere Auswertung des Temperaturverlaufs 50 ist dabei die Gelierzeitpunkttemperatur, die bei dem in Fig. 3 dargestellten Ausführungsbeispiel etwa 170° C beträgt.

Wie man in Fig. 3 erkennt, steigt die Temperatur des Harzes nach dem Erreichen des Gelierpunktes 52 weiter an und erreicht schließlich eine in Fig. 3 mit dem Bezugszeichen 53 versehene maximale Exothermiespitze E.

Zeichnet man durch den Minimaltemperaturpunkt 51 eine Gerade parallel zur Zeitachse der Fig. 3 und parallel zur Temperaturachse Geraden durch den Gelierpunkt 52 und die Exothermiespitze 53, so kann die durch diese Geraden begrenzte Fläche unter der Kurve des Temperaturverlaufs 50 der jeweils freigewordenen Energie bis zum Gelierzeitpunkt 52 bzw. der freigewordenen Energie bis zum Erreichen der Exothermiespitze 53 zugeordnet werden. Die gesamte freigewordene Wärmeenergie zwischen dem Zeitpunkt der Minimaltemperatur 51 und dem Zeitpunkt der Exothermiespitze 53 läßt sich somit der Fläche zuordnen, die einerseits durch die Parallele 54 zur Zeitachse und andererseits durch die Parallele 55 zur Temperaturachse sowie durch die Kurve zum Temperaturverlauf 50 begrenzt ist. Je nach der Gestalt des Temperaturverlaufs 50, d.h. entsprechend dem Abstand zwischen der Minimaltemperatur 51 und der Exothermiespitze 53 sowie der benötigten Zeitdauer zwischen diesen beiden Temperaturen, hat die Grundfläche 56, die in der oben beschriebenen Weise ermittelt werden kann, unterschiedliche Werte.

Für jeden der von den Thermoelementen 21 bis 26 erfaßten Temperaturverläufe 41 bis 46 wird entsprechend dem als Beispiel diskutierten Temperaturverlauf 50 während der Kalibriermessung die Grundfläche 56 bei unterschiedlichen Kalibrierformtemperaturen für ein gegebenes Harzsystem bestimmt.

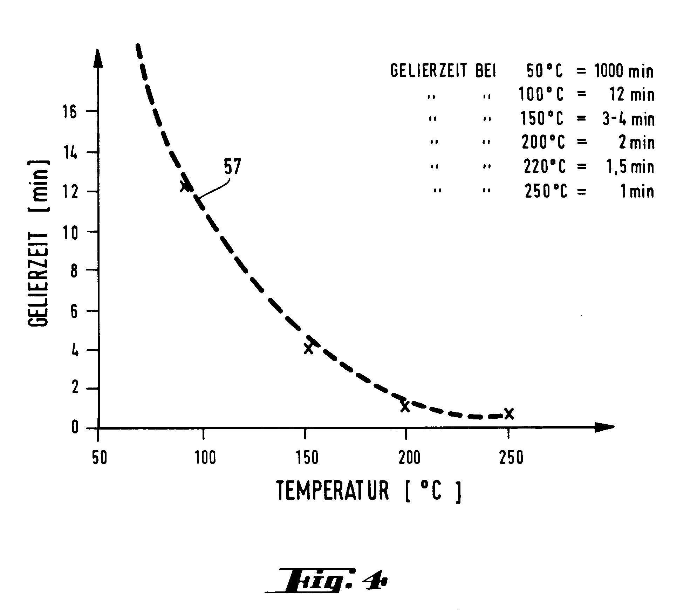

Fig. 4 veranschaulicht in einer Gelierzeitkurve 57 für ein beispielsweise verwendetes Harzsystem wie die Gelierzeit in Minuten bei steigender Temperatur des Harzsystems bzw. der Kalibrierform 1 abnimmt. Bei einer Harztemperatur von 100° C beträgt die Gelierzeit beispielsweise 12 Minuten, bei 150° C etwa 4 Minuten, bei 200° C etwa 2 Minuten und bei 250° C etwa 1 Minute.

Gemäß der vorliegenden Erfindung wird ausgehend von dem Temperaturverlauf 50 eine skalare Größe oder Masszahl bestimmt, indem das Kalibriervolumen eines Vergleichskörpers bestimmt wird, dessen Grundfläche die in Fig. 3 dargestellte Grundfläche 56 ist und dessen in Richtung der Temperaturachse zunehmende Höhe dem Kehrwert der in Fig. 4 dargestellten Gelierzeitkurve 57 entspricht. Dies bedeutet eine Gewichtung der Grundfläche 56 gemäß der Reaktivität bzw. Gelierzeit eines Harzes in Funktion der Temperatur.

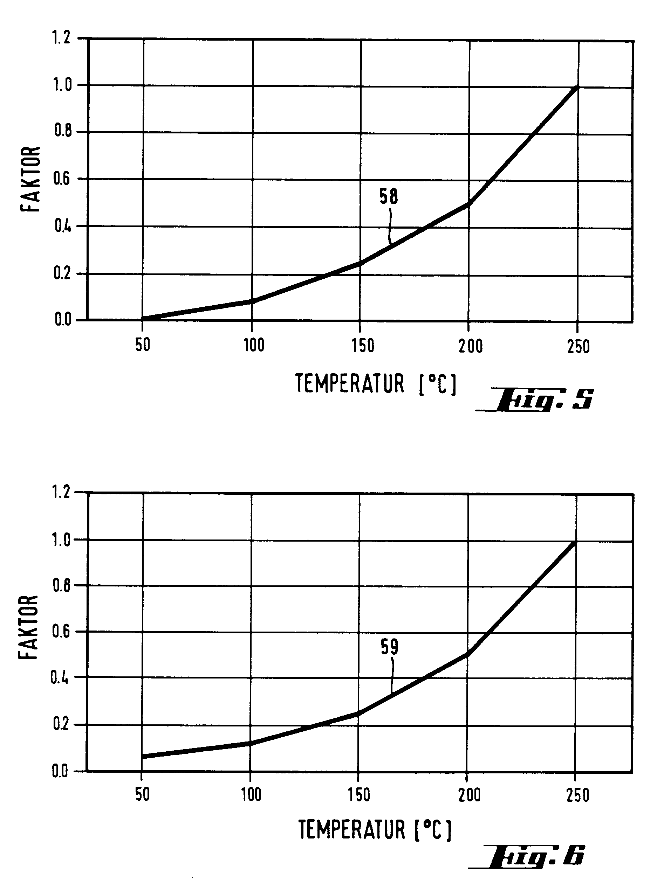

Fig. 5 zeigt eine Gelierfaktorkurve 58, die durch Bilden des Kehrwertes aus der Gelierzeitkurve 57 gewonnen ist. Die Gelierfaktorkurve 58 stellt die Höhe des Vergleichskörpers über der Grundfläche 56 in Fig. 3 entlang der Temperaturachse dar. Das Kalibriervolumen des auf der Grundfläche 56 gebildeten Vergleichskörpers hängt somit wegen der dort geringeren Höhe nur wenig vom Temperaturverlauf 50 bei niedrigen Temperaturen, aber sehr stark vom Temperaturverlauf 50 bei höheren Temperaturen ab, da in diesem Bereich der Vergleichskörper in Richtung der rechtwinklig zur Temperaturachse und Zeitdauerachse in Fig. 3 verlaufenden Achse sehr hoch ist. Diese Höhe ist durch den Verlauf der Gelierfaktorkurve 58 in Fig. 5 wiedergegeben.

Die obigen Ausführungen zeigen, daß es mit Hilfe eines Computerprogramms ohne weiteres möglich ist, ausgehend von einem Temperaturverlauf 50, der beispielhaft für einen der Temperaturverläufe 41 bis 46 steht, eine skalare Größe zu bestimmen, die als Kalibriervolumen eines einem Temperaturverlauf 50 zugeordneten Vergleichskörpers bezeichnet worden ist.

Gemäß der Erfindung wird für ein spezielles Harzsystem bei einer Vielzahl von Temperaturen der Kalibrierform 1 mit Hilfe der Thermoelemente 21 bis 26 an einer Vielzahl von unterschiedlichen Volumenelementen eine Vielzahl von Temperaturverläufen 50 gewonnen, die in der Weise ausgewertet werden, daß ein Zeitintegral über die Temperatur eine Grundfläche 56 bildet. In einem weiteren Schritt wird oberhalb der Grundfläche 56 entsprechend der durch die Gelierfaktorkurve 58 in Fig. 5 veranschaulichten Kurve eine in Richtung der Temperaturachse der Fig. 3 gewölbte Fläche aufgespannt, so daß diese Fläche oberhalb der Grundfläche 56 einen Vergleichskörper begrenzt, dessen Kalibriervolumen der gesamten freigewordenen Wärmeenergie zugeordnet werden kann.

Während der Kalibriermessung werden in der oben beschriebenen Weise eine Vielzahl von Vergleichskörper-Kalibriervolumen bestimmt. Jedes Kalibriervolumen stellt dabei eine skalare Größe dar, die den dem Temperaturverlauf 50 jeweils zugeordneten Vergleichskörper charakterisiert. Jedem der so bestimmten Vergleichskörper-Kalibriervolumen ist weiterhin die durch die Gelierpunktsonde markierte Temperatur des Gelierpunktes 52 zugeordnet. Der die in der Kalibrierform 1 gewonnenen Daten auswertende Computer erzeugt somit für jedes einer Kalibrierung unterzogene Harzsystem eine Liste mit Zwei Spalten, wobei in der ersten Spalte die Kalibriervolumen aller erfaßten Temperaturverläufe 50 aufgeführt sind und wobei in der zweiten Spalte jeweils die zugeordneten Gelierzeitpunkttemperaturen als zweite skalare Größe von Kalibrierpaaren erfaßt ist.

Der vorliegenden Erfindung liegt die Erkenntnis zugrunde, daß es möglich ist, in der oben beschriebenen Weise charakteristische skalare Größen zu bilden, die als Kalibriervolumen von Vergleichskörpern bezeichnet worden sind, denen jeweils Gelierzeitpunkttemperaturen in eindeutiger Weise zugeordnet werden können. Wenn bei einem gegebenen Harzsystem aufgrund einer Kalibriermessung die oben erwähnten Zahlenpaare Kalibriervolumen - Gelierzeitpunkttemperatur erfaßt sind, kann aufgrund einer Messung des Temperaturverlaufs des Harzes innerhalb einer anderen Form aufgrund der oben erwähnten Tabelle die Gelierzeitpunkttemperatur bestimmt werden, indem der Temperaturverlauf 50 zwischen der Minimaltemperatur 51 und der Exothermiespitze 53 erfaßt wird und aus diesem Temperaturverlauf 50 analog der oben erwähnten Weise das Meßvolumen eines Vergleichskörpers ermittelt wird. Wenn das auf diese Weise ermittelte Vergleichskörpervolumen in der während der Kalibriermessungen angelegten Tabelle vorkommt, gibt diese Tabelle sofort Aufschluß über die Gelierzeitpunkttemperatur.

Bei einem rechnerisch etwas vereinfachten Verfahren wird statt des in Fig. 5 dargestellten Verlaufs der Gelierfaktorkurve 58 eine Gelierfaktorkurve 59 verwendet, die rechnerisch dadurch bestimmt worden ist, daß für jedes Grad Temperaturerhöhung der Gelierfaktor um 1,395947979 % erhöht worden ist, wobei der Gelierzeitfaktor für 1° C 3,16862337434·10⁻² beträgt. Eine derartige Berechnung ergibt für 50° C einen Gelierzeitfaktor von 0,0625, für 100° C einen Gelierzeitfaktor von 0,125, für 150° C einen Gelierzeitfaktor von 0,25, für 200° C einen Gelierzeitfaktor von 0,5 und für 250° C einen Gelierzeitfaktor von 1. Diese Faktoren ergeben wie bereits erwähnt die Höhe des Vergleichskörpers über der Grundfläche 56 in Fig. 3 in Richtung einer Koordinatenachse rechtwinklig zu den in Fig. 3 dargestellten Achsen an.

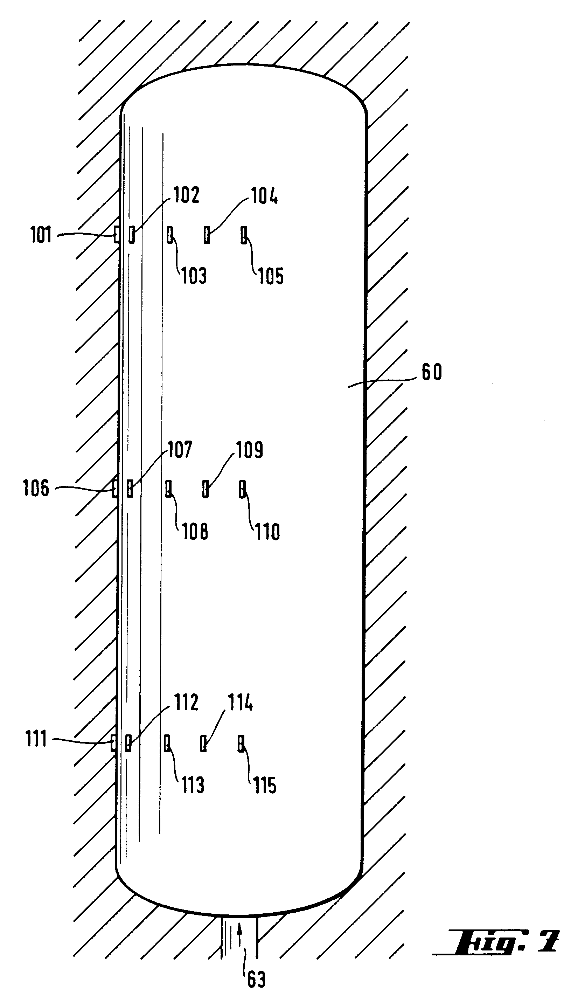

Wenn die oben erörterten Kalibriermessungen für ein Harzsystem abgeschlossen sind, das dazu verwendet werden soll, in einer Produktionsform ein Formteil, beispielsweise ein Elektro-Isolierteil im Druckgelierverfahren herzustellen, kann eine Produktionsform verwendet werden, die beispielsweise die in Fig. 7 dargestellte Gießform hat. Die Erfindung gestattet es, das Fortschreiten der Gelierfront in einer in Fig. 7 als Beispiel dargestellten Produktionsform zu messen, indem lediglich Temperaturverläufe 50 der in Fig. 3 dargestellten Art erfaßt und ausgewertet werden. Mechanische Gelierzeitpunktsonden, wie sie bei der Kalibriermessung in der Kalibrierform verwendet wurden, sind bei einer Überprüfungsmessung unter Produktionsbedingungen in der vorgesehenen Produktionsform nicht erforderlich.

Fig. 7 zeigt schematisch einen Querschnitt durch eine zylindrische Produktionsform 60, die beispielsweise einen Durchmesser von 60 mm und eine Länge von 180 mm hat. An den durch kurze Striche gekennzeichneten Stellen sind in der Produktionsform 60 eine Vielzahl von räumlich verteilten Thermoelementen 102 bis 115 entsprechend den Thermoelementen 21 bis 26 angeordnet.

Wie man in Fig. 7 erkennen kann, sind die Thermoelemente 102 bis 105 in einer oberen Ebene, die Thermoelemente 107 bis 110 und die Thermoelemente 112 bis 115 in einer unteren Ebene angeordnet. Zusätzlich befindet sich in jeder Ebene noch ein weiteres Thermoelement 101,106,111, das es gestattet, die Temperatur unmittelbar an der Wand der Produktionsform 60 zu erfassen.

In Fig. 7 erkennt man unten einen Anguß 63, durch den zur Durchführung einer Überprüfungsmessung entsprechend dem Druckgelierverfahren ein Harzsystem eingebracht werden kann, für das vorher Kalibriermessungen durchgeführt worden sind. Sobald die Produktionsform 60 mit Harz gefüllt ist, werden an den Thermoelementen 102 bis 115 Temperaturverläufe entsprechend dem Temperaturverlauf 50 erfaßt und über in der Zeichnung nicht dargestellte Leitungen an einen Computer gegeben, der zunächst alle von den Thermoelementen 102 bis 115 gelieferten Temperaturverläufe 50 erfaßt. Ein im Computer gespeichertes Programm errechnet nach dem Vorliegen der Temperaturverläufe 50 zwischen der jeweiligen Minimaltemperatur 51 und der jeweiligen Exothermiespitze 53, das als Meßvolumen bezeichenbare Volumen eines Vergleichskörpers mit der Grundfläche 56 und einer Höhe, die in der oben beschriebenen Weise durch die inversen Gelierzeiten definiert ist.

Wenn fürjeden Temperaturverlauf 50, der von den Thermoelementen 102 bis 115 erfaßt worden ist, die als Meßvolumen eines Vergleichskörpers bezeichnete skalare Größe bestimmt ist, ist es dem Computer auf einfache Weise möglich, die Gelierzeitpunkttemperatur an den Stellen der Thermoelemente 102 bis 115 zu bestimmen. Dies geschieht in einfacher Weise dadurch, daß die bei der Überprüfungsmessung der Produktionsform 60 bestimmten Meßvolumen der Vergleichskörper mit den vorher bestimmten Kalibriervolumen verglichen werden, die im Speicher des Computers als Tabelle abgelegt sind. Im einfachsten Fall wird sich ergeben, daß die skalare Größe des Meßvolumens als Kalibriervolumen in der Tabelle des Computers vorkommt. Die zugeordnete Gelierzeitpunkttemperatur ergibt sich unmittelbar aus der dem abgespeicherten Kalibriervolumen zugeordneten Temperatur. Fällt der Wert eines bei der Überprüfungsmessung einer Produktionsform 60 bestimmten Meßvolumens nicht mit einem im Speicher des Rechners vorhandenen Kalibriervolumen zusammen, so wird mit Hilfe des Rechners eine lineare Interpolation durchgeführt, indem der Rechner die Kalibriervolumen oberhalb und unterhalb des beim Überprüfen der Produktionsform 60 bestimmten Meßvolumens heranzieht, um aus den beiden den benachbarten Kalibriervolumen zugeordneten Gelierzeitpunkttemperaturen eine Gelierzeitpunkttemperatur zu berechnen, die einen Interpolationswert für das zwischen zwei Kalibriervolumen fallende Meßvolumen darstellt.

Auf diese Weise gestattet es die Erfindung, jedem durch die Thermoelemente 102 bis 115 erfaßten Temperaturverlauf 50 zunächst einen Vergleichskörper mit einem Meßvolumen zuzuordnen und anschließend aus der Tabelle der Kalibriervolumen und Gelierzeitpunkttemperaturen eine direkte oder interpolierte Gelierzeitpunkttemperatur zu entnehmen, die für das Volumenelement in der Nähe des jeweiligen Thermoelementes 102 bis 115 gilt. Bei einer graphischen Ausgabe der in der Produktionsform gemessenen Temperaturverläufe ist es daher möglich, für jeden dieser Temperaturverläufe 50 die Gelierzeitpunkttemperatur anzuzeigen.

Durch Auswerten der Temperaturverläufe 50 für die Thermoelemente 102 bis 115 ist es somit einerseits möglich am Ort jeden Thermoelementes ein zeitliches Temperaturprofil mit einer Markierung des Gelierpunktes zu erstellen. Weiterhin ist es infolge der Kenntnis der jeweiligen Temperatur an den Thermoelementen 102 bis 115 möglich, den räumlichen Temperaturverlauf und Isothermen für eine Produktionsform darzustellen. Die Genauigkeit der Isothermen ist dabei um so höher, je zahlreicher und je geschickter die Thermoelemente 102 bis 115 in der Produktionsform verteilt sind.

Da für das Harzvolumen, das jeweils eines der Thermoelemente 102 bis 115 umgibt, in der oben beschriebenen Weise durch Nachschlagen in der Tabelle der Kalibiervolumen und zugeordneten Gelierzeitpunkttemperaturen über die jeweils erfaßten Meßvolumen eine Bestimmung der Gelierzeitpunkttemperaturen möglich ist, gestattet es die Erfindung, nicht nur zu verschiedenen Zeiten den Verlauf der Isothermen innerhalb der Produktionsform anzugeben, sondern auch den Verlauf der Gelierfront zu verschiedenen Zeiten während des Aushärtungsvorgangs. Ist im Bereich eines Thermoelementes 102 bis 115 die individuelle in der oben beschriebenen Weise durch Auswerten des Temperaturverlaufs 50 bestimmte Gelierzeitpunkttemperatur gerade erreicht, liegt dieses Thermoelement 102 bis 115 gerade in der Gelierfront. Ist die Temperatur höher, ist die Gelierfront bereits durchgezogen. Liegt die Temperatur kleiner, befindet sich das jeweilige Thermoelement 102 bis 115 noch in nicht ausgehärtetem Harz.

Dem Computer zur Auswertung der von den Thermoelementen 102 bis 115 gelieferten Temperaturverläufe 50 ist eine graphische Anzeigeeinheit zugeordnet, die es gestattet, den Umriß der Produktionsform sowie die Lage der jeweils verwendeten Thermoelemente 102 bis 115 darzustellen. Zusätzlich zur Anzeige der Lage der Thermoelemente 102 bis 115 gestattet es die graphische Anzeigeeinheit, durch Farben und Isothermen die thermische Verteilung entlang einem Schnitt durch die Produktionsform darzustellen. Außerdem gestattet es die graphische Anzeigeeinheit, den Verlauf der Gelierfront in Funktion der Zeit sowie den Verlauf der Exothermiefront in Funktion der Zeit anzuzeigen.

Da das Fortschreiten der Gelierfronten in der Produktionsform das entscheidende Kriterium für die Qualität eines Gießteils ist, ist man daran interessiert, zu rasch laufende, ungleichmäßig verlaufende oder verschmelzende Gelierfronten zu vermeiden, weil sie zu lokalen Exothermiespitzen, Lunkerbildungen und Spannungzentren führen. Dank der Erfindung können die Verläufe der Gelierfronten räumlich erfaßt und dargestellt werden. Sind sie ungünstig, so können diese durch Ändern verschiedener Parameter beeinflußt werden. Bei diesen Parametern handelt es sich um die Temperatur des Harzsystemes, die Temperatur der Produktionsform und die Reaktivität der Gießharzmasse.

Das vorstehend beschriebene, erfindungsgemässe Verfahren ist besonders für den Einsatz bei der Herstellung von grossvolumigen Giesslingen gemäss dem Druckgelierverfahren geeignet, jedoch keineswegs darauf beschränkt. So lässt es sich u.a. auch hervorragend im Zusammenhang mit der z.B. in der EP-A-0333456 beschriebenen Herstellung von dünnwandigen Giesslingen relativ grosser Ausdehnung einsetzen.

## Patentansprüche

1. Verfahren zum Überprüfen des Gelierprozesses nach dem Gießen eines reaktiven Harzsystems in eine Produktionsform, dadurch gekennzeichnet, daß während einer Kalibriermessung das Gelierverhalten des für die Herstellung des Gießlings vorgesehenen Harzsystems in einer auf verschiedene vorherbestimmte Temperaturen aufheizbaren Kalibrierform (1) ermittelt wird, indem bei verschiedenen Kalibrierformtemperaturen für mehrere unterschiedliche Gelierverhalten aufweisende Volumenelemente innerhalb der Kalibrierform (1) nach dem Einbringen des Harzsystems in die Kalibrierform (1) mittels Gelierpunktsonden (31-36) der Gelierzeitpunkt und mittels Thermofühler (21-26) die zugeordneten zeitlichen Temperaturverläufe vom Zeitpunkt des Einbringens des Harzsystems in die Kalibrierform (1) bis zur jeweiligen nach dem Gelierzeitpunkt auftretenden maximalen Temperatur bei der Exothermiespitze erfaßt werden, daß die ermittelten jeweiligen Gelierzeitpunkttemperaturen abgespeichert werden und für jeden Temperaturverlauf eine zugeordnete charakteristische skalare Größe bestimmt wird, indem das Kalibriervolumen eines zugeordneten Vergleichskörpers ermittelt und gespeichert wird, dessen Grundfläche durch den zeitlichen Temperaturverlauf zwischen der minimalen und der maximalen Temperatur des Harzsystems in der Kalibrierform und dessen Höhe durch einen sich in Richtung der Temperaturachse des Temperaturverlaufs verändernden Gelierfaktor bestimmt ist, der dem Kehrwert der Gelierzeit bei der jeweiligen Temperatur entspricht, daß während einer Überprüfungsmessung unter Produktionsbedingungen in der vorgesehenen Produktionsform durch eingießbare Thermofühler die zeitlichen Temperaturverläufe des bei der Kalibrierung verwendeten Harzsystems an mehreren unterschiedlichen Meßstellen innerhalb der Produktionsform erfaßt und gespeichert werden, daß für die so ermittelten Temperaturverläufe zwischen den minimalen und maximalen Temperaturen des Harzsystems an den jeweiligen Meßstellen die Meßvolumen zugeordneter Vergleichskörper ermittelt werden, deren Grundfläche durch den zeitlichen Temperaturverlauf zwischen der minimalen und der maximalen Temperatur des Harzsystems in der Produktionsform und dessen Höhe durch den Gelierzeitfaktor bestimmt ist, daß die für die unterschiedlichen Meßstellen innerhalb der Produktionsform ermittelten Meßvolumen der Vergleichskörper mit den zusammen mit den Gelierzeitpunkttemperaturen abgespeicherten Kalibriervolumen der Vergleichskörper verglichen werden und daß für die unterschiedlichen Meßstellen in der überprüften Produktionsform durch eine Interpolation der Meßvolumen zwischen den gespeicherten Kalibriervolumen die zugeordneten interpolierten individuellen Gelierzeitpunkttemperaturen ermittelt werden, aus denen sich ausgehend von den gespeicherten räumlich verteilt erfaßten zeitlichen Temperaturverläufen und dem sich aus diesen ergebenden Verlauf der Isothermen für jeden Zeitpunkt der Verlauf der die Isothermen schneidenden Gelierfront in der Produktionsform ergibt.

2. Verfahren nach Anspruch 1, dadurch gekennzeichnet, daß bei der Kalibriermessung die Härtung des Harzsystems ohne Nachdruck ausgeführt wird.

3. Verfahren nach Anspruch 1 oder 2, dadurch gekennzeichnet, daß bei der Erfassung der zeitlichen Temperaturverläufe an den Meßstellen innerhalb der Produktionsform nach dem Druckgelierverfahren eine Härtung mit Nachdruck ausgeführt wird.

4. Verfahren nach Anspruch 1, dadurch gekennzeichnet, daß aus den räumlich verteilt erfaßten zeitlichen Temperaturverläufen der Verlauf der Exothermiefront in Funktion der Zeit bestimmt wird.

5. Verfahren nach Anspruch 1, dadurch gekennzeichnet, daß eine Kalibrierform mit einer rotationssymmetrischen Formkavität verwendet wird.

6. Anwendung des Verfahrens nach einem der vorangehenden Ansprüche bei der Herstellung von grossvolumigen Giesslingen insbesondere nach dem Druckgelierverfahren.

7. Anwendung des Verfahrens nach einem der Ansprüche 1-5 bei der Herstellung von dünnwandigen Giesslingen.

## Claims

1. A method of checking the gelling process after pouring a reactive resin system into a production mould, which comprises ascertaining, in a calibrating mould (1) that can be heated to various predetermined temperatures, during a calibration measurement, the gelling behaviour of said resin system provided for the manufacture of a casting by detecting at various calibrating mould temperatures, after introducing said resin system into said calibrating mould (1) and using gelling point probes (31-36), the moment of gelling of several volume elements which have different gelling behaviours and are located inside said calibrating mould (1) and by detecting, using temperature sensors (21-26), the associated temperature patterns over time from the moment at which said resin system is introduced into said calibrating mould (1) up to the maximum temperature occurring at the peak of the exothermic reaction after the moment of gelling, storing the determined gelling moment temperatures and establishing an associated characteristic scalar value for each temperature pattern by determining and storing the calibration volume of an associated comparison body the base of which is established by the temperature pattern over time between the minimum and the maximum temperature of said resin system in said calibrating mould, and the height of which is established by a gelling factor that changes in the direction of the temperature axis of said temperature pattern, which factor corresponds to the reciprocal of the gelling time at the temperature concerned, detecting and storing during a checking measurement, at several different measuring sites inside the production mould, under production conditions in said production mould provided and using embeddable temperature sensors, the temperature patterns over time of said resin system used in the calibration, determining for the so determined temperature patterns between the minimum and the maximum temperatures of said resin system, at the particular measuring sites, the measured volumes of associated comparison bodies, the base of which is established by the temperature pattern over time between the minimum and the maximum temperature of said resin system in said production mould and the height of which is established by the gelling time factor, comparing the measured volumes of said comparison bodies, which volumes are determined for the different measuring sites inside said production mould, with said calibration volumes of said comparison bodies, which volumes are stored together with the gelling moment temperatures, and determining for the different measuring sites in said checked production mould, by interpolating said measured volumes between said stored calibration volumes, the associated interpolated individual gelling moment temperatures, from which, on the basis of the stored temperature patterns over time detected in a spatially distributed manner, and on the basis of the pattern of the isotherms resulting therefrom, the progress of the gelling front in said production mould, which front intersects said isotherms, is deduced for each point in time.

2. A method according to claim 1, wherein, during said calibration measurement, the hardening of said resin system is carried out without excess pressure.

3. A method according to claim 1 or claim 2, wherein, during the detection of said temperature patterns over time at said measuring sites inside said production mould, a hardening is carried out with excess pressure in accordance with the pressure gelling process.

4. A method according to claim 1, wherein the progress of the exothermic front as a function of time is established from said temperature patterns over time detected in a spatially distributed manner.

5. A method according to claim 1, wherein a calibrating mould having a rotationally symmetrical mould cavity is used.

6. The use of the method according to any one of the preceding claims in the manufacture of large-volume castings especially in accordance with the pressure gelling process.

7. The use of the method according to any one of claims 1 to 5 in the manufacture of thin-walled castings.

## Revendications

1. Procédé de contrôle de la gélification après le coulage d'un système réactif générateur de résine ("système de résine réactive") dans un moule de production, procédé caractérisé en ce que
- pendant une mesure d'étalonnage, on recherche l'évolution de la gélification du système générateur de la résine destinée à fabriquer des pièces moulées, dans un moule d'étalonnage (1) qui peut étre porté à des températures différentes, en relevant, à diverses températures de ce moule d'étalonnage (1),l'instant de la gélification d'un certain nombre d'éléments de volume dans lesquels l'évolution de la gélification est différente, après l'introduction du système générateur de résine dans ce moule et en relevant au moyen de capteurs thermiques (21-26) les variations correspondantes de la température depuis l'instant où le système générateur de résine a été introduit dans le moule jusqu'à la température maximale au sommet de la courbe d'exothermie,
- on mémorise les diverses températures relevées au moment de la gélification et l'on détermine pour chaque courbe de températures une grandeur scalaire caractéristique, en relevant et mémorisant le volume-étalon d'un corps-étalon associé, dont la surface de base est déterminée par l'évolution dans le temps, entre les valeurs minimale et maximale, de la température du système générateur de résine dans le moule et dont la hauteur est déterminée par un facteur de gélification, qui varie dans le sens de l'axe des températures de la courbe des températures et qui correspond à l'inverse de la durée de gélification à la température respective,
- pendant une mesure de contrôle dans les conditions de production dans le moule de production prévu, on relève, au moyen de capteurs thermiques plongeants, les variations, dans le temps, de la température du système générateur de résine utilisé au cours de l'étalonnage en plusieurs emplacements différents dans le moule, et on les mémorise,
- on établit, pour les variations entre les valeurs minimale et maximale de la température de la résine à ces emplacements, le volume mesuré de corps-étalons associés, dont la surface de base est déterminée au moyen de la variation de la température du système générateur de résine dans le moule de production entre ces valeurs minimale et maximale et dont la hauteur est déterminée par l'entremise du facteur de durée de gélification,
- on compare les volumes des corps-étalons aux divers emplacements de mesure dans le moule de production, aux volumes-étalons de ces corps, mémorisés avec les températures de gélification, et
- on établit pour les divers emplacements de mesure dans le moule de production contrôlé, par interpolation des volumes mesurés entre les volumes-étalons mémorisés, les températures individuelles de gélification interpolées associées, au moyen desquelles on obtient, en partant des courbes de température relevées localement et mémorisées et de l'allure des isothermes qui en résulte, l'évolution à chaque instant du front de gélification qui coupe ces isothermes dans le moule de production.

2. Procédé selon la revendication 1, caractérisé en ce que, au cours de la mesure d'étalonnage, le durcissement du système générateur de la résine est réalisé sans compression complémentaire.

3. Procédé selon la revendication 1 ou 2, caractérisé en ce que, pendant le relevé des courbes de température aux emplacements de mesure dans le moule de production, on exécute un durcissement avec compression complémentaire après la gélification sous pression.

4. Procédé selon la revendication 1, caractérisé en ce que l'on détermine l'évolution du front d'exothermie en fonction du temps à partir des courbes de températures locales relevées en fonction du temps.

5. Procédé selon la revendication 1, caractérisé en ce que l'on utilise un moule d'étalonnage comportant une cavité ayant une forme ou symétrie de révolution.

6. Application du procédé selon l'une des revendications précédentes à la fabrication de pièces moulées volumineuses, obtenues notamment par gélification sous pression.

7. Application du procédé selon l'une des revendications 1 à 5 à la fabrication de pièces moulées à paroi mince.
